(19)

Europäisches
Patentamt
European
Patent Office
Office européen
des brevets

(11) **EP 4 108 649 A1**

(12) **EUROPEAN PATENT APPLICATION**
published in accordance with Art. 153(4) EPC

(43) Date of publication:
**28.12.2022 Bulletin 2022/52**

(21) Application number: **21757274.2**

(22) Date of filing: **15.02.2021**

(51) International Patent Classification (IPC):
*C07C 4/00* (2006.01)    *B01J 29/40* (2006.01)
*C01B 39/38* (2006.01)    *C07B 61/00* (2006.01)
*C07C 11/04* (2006.01)    *C07C 11/06* (2006.01)

(52) Cooperative Patent Classification (CPC):
**B01J 29/40; C01B 39/38; C07B 61/00; C07C 4/00;
C07C 11/04; C07C 11/06;** Y02P 20/52

(86) International application number:
**PCT/JP2021/005557**

(87) International publication number:
**WO 2021/166854 (26.08.2021 Gazette 2021/34)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **21.02.2020 JP 2020027815**

(71) Applicants:
• **SUMITOMO CHEMICAL COMPANY, LIMITED
Tokyo 103-6020 (JP)**
• **Muroran Institute of Technology
Muroran-shi
Hokkaido 050-8585 (JP)**

(72) Inventors:
• **UEMICHI, Yoshio
Muroran-shi
Hokkaido 050-8585 (JP)**
• **KANDA, Yasuharu
Muroran-shi
Hokkaido 050-8585 (JP)**
• **OKAMOTO, Yuki
Ichihara-shi
Chiba 299-0195 (JP)**

(74) Representative: **Vossius & Partner
Patentanwälte Rechtsanwälte mbB
Siebertstraße 3
81675 München (DE)**

(54) **METHOD FOR PRODUCING OLEFINS**

(57) To provide a method for producing olefins having the carbon atom number of 2 to 3 with a high yield. A method for producing olefins including following steps (1) and (2): step (1): heating a polyolefin plastic to obtain a decomposed product (pyrolysis step); and step (2): causing the decomposed product obtained at the step (1) to contact with an MFI zeolite containing sodium atoms in the range of 0.10% by mass to 0.30% by mass to obtain catalytically cracked products containing olefins (catalytic cracking step).

**EP 4 108 649 A1**

## Description

### TECHNICAL FIELD

The present invention relates to a method for producing olefins.

### BACKGROUND ART

The key raw materials of a petrochemical industry are lower olefins such as ethylene and propylene, as well as aromatic hydrocarbons such as benzene, toluene, and xylene, obtained by cracking and reforming of a naphtha. From these starting raw materials, a wide variety of chemicals are synthesized. Among these, plastics are produced most with a large production mass, and they are widely used from industrial products to daily goods because of the excellent characteristics as a material, such as a light weight, a corrosion resistance, and a flexibility in the molding thereof. As a result, the amount of the wasted plastics is also enormous.

Non Patent Literature 1 describes a chemical recycling technology that can efficiently crack polyethylene catalytically into a petrochemical feedstock. Specifically, the method is described in which an MFI zeolite containing sodium atoms is used to catalytically crack polyethylene to obtain the olefins having the carbon atom number of 2 to 5.

### RELATED ART DOCUMENTS

Non Patent Literature

Non Patent Literature 1: Fine Chemicals Monthly (December 2017, Vol. 46, No. 12)

### DISCLOSURE OF INVENTION

### PROBLEM TO BE SOLVED BY THE INVENTION

However, the method described in Non Patent literature 1 is not necessarily satisfactory in the yield of the resulting olefins having the carbon atom number of 2 to 3. The present invention provides a method for producing olefins having the carbon atom number of 2 to 3 with a high yield.

### MEANS FOR SOLVING PROBLEM

The present invention provides the following [1] to [8].

[1] A method for producing olefins comprising following steps (1) and (2):

step (1): heating a polyolefin plastic to obtain a decomposed product (pyrolysis step); and
step (2): causing the decomposed product obtained at the step (1) to contact with an MFI zeolite containing sodium atoms in the range of 0.10% by mass to 0.30% by mass to obtain catalytically cracked products containing olefins (catalytic cracking step).

[2] The method for producing olefins according to above [1], wherein the olefins are olefins having the carbon atom number of 2 to 3.
[3] The method for producing olefins according to above [1] or [2], wherein a ratio of a mole number of silicon atoms to a mole number of aluminum atoms in the MFI zeolite is 80 to 250.
[4] The method for producing olefins according to any one of above [1] to [3], wherein a pyrolysis temperature $T^1$ at the step (1) is 400°C to 500°C.
[5] The method for producing olefins according to any one of above [1] to [4], wherein a contact temperature $T^2$ at the step (2) is 450°C to 600°C.
[6] The method for producing olefins according to any one of above [1] to [5], wherein a pyrolysis temperature $T^1$ at the step (1) and a contact temperature $T^2$ at the step (2) satisfy a following equation:

$$0°C \leq T^2 - T^1 \leq 200°C.$$

[7] The method for producing olefins according to any one of above [1] to [6], wherein the polyolefin plastic is at least one plastic selected from the group consisting of polyethylene and polypropylene.

[8] The method for producing olefins according to above [7], wherein the polyolefin plastic is a mixture of polyethylene and polypropylene.

**EFFECT OF THE INVENTION**

According to the present invention, the method for producing olefins having the carbon atom number of 2 to 3 with a high yield can be provided. In addition, the present invention can provide the method for producing the olefins in which the ratio of the olefins having the carbon atom number of 2 to 3 to paraffins (hereinafter this ratio is referred to as olefin/paraffin ratio) contained in the catalytic cracking is excellent.

**DESCRIPTION OF EMBODIMENTS**

The present invention is a method for producing olefins comprising the following steps (1) and (2):

step (1): heating a polyolefin plastic to obtain a decomposed product (pyrolysis step), and

step (2): causing the decomposed product obtained at the step (1) to contact with an MFI zeolite containing sodium atoms in the range of 0.10% by mass to 0.30% by mass to obtain catalytically cracked products containing olefins (catalytic cracking step).

<Step (1)>

Polyolefin Plastics

Examples of the polyolefin plastics may include polyethylene, polypropylene, polybutene, an ethylene-vinyl acetate copolymer, and an ethylene-$\alpha$-olefin copolymer. Among these, polyethylene, polypropylene, and a mixture of polyethylene and polypropylene are preferable.

The polyolefin plastics may also include an industrial product such as a molded article made from the above polyolefin plastics. Examples of the industrial product may include plastic containers and packaging collected under the Containers and Packaging Recycling Law. In addition to the polyolefin plastics discribed above, these industrial products may usually include polystyrene, polyethylene terephthalate (PET), polyvinyl chloride (PVC), polyamide, polycarbonate, polyurethane, polyester, and natural and synthetic rubbers. However, these are removed by a usual sorting and prosessing method so as to carry out the method for producing olefins according to the present invention.

• Pyrolysis step

Pyrolysis temperature at the step (1): $T^1$ is usually in the range of 350°C to 550°C, and preferably in the range of 400°C to 500°C.

Pyrolysis pressure at the step (1): $P^1$ is usually in the range of 0 MPaG to 5 MPaG, and preferably in the range of 0 MPaG to 0.5 MPaG.

In the pyrolysis at the step (1), a water vapor, or an inert gas such as nitrogen gas or $CO_2$ gas may coexist.

Pyrolysis at the step (1) can be carried out in a reaction vessel usually made of quartz glass, carbon steel, stainless steel, or the like.

The decomposed product obtained at the step (1) is usually hydrocarbons having the carbon atom number of about 1 to 50, or hydrogen.

<Step (2)>

• MFI Zeolite

The MFI zeolite used in the embodiment contains 0.10% by mass to 0.30% by mass of sodium atoms. The content of the sodium atoms is preferably in the range of 0.12% by mass to 0.28% by mass, and more preferably in the range of 0.15% by mass to 0.25% by mass.

The MFI zeolite used in the embodiment usually contains silicon atoms, aluminum atoms, oxygen atoms, and hydrogen atoms as the atoms other than the sodium atoms.

The MFI zeolite means the crystalline aluminosilicate having the MFI structure according to the IZA (International Zeolite Association) structure code. Specifically, examples of the MFI zeolite may include $H^+$-ZSM-5, $NH_4^+$-ZSM-5, $Na^+$-ZSM-5, and $Ca^{2+}$-ZSM-5.

The MFI zeolite used in the embodiment can be prepared by introducing sodium atoms after H⁺-ZSM-5 is prepared in the usual way. The MFI zeolite used in the embodiment may also be prepared by introducing sodium atoms into a commercially purchased H⁺-ZSM-5.

Hereinafter, the production method of the MFI zeolite used in the embodiment will be described.

The MFI zeolite used in the embodiment may be produced by the production method that includes a step of crystallizing a mixture containing a silicon source, an aluminum source, a templating agent, and an alkali metal source to obtain a ZSM-5 zeolite. The term "templating agent" refers to the substance that forms a porous structure in a crystalline aluminosilicate.

As for the silicon source, a conventionally known silica-containing material used for production of various zeolites may be used. Specifically, examples of the silica-containing material that can be used may include a tetraethyl orthosilicate, a colloidal silica, a silica gel dry powder, and a silica hydrogel.

As for the aluminum source, a conventionally known aluminum source used for production of various zeolites may be used. Specifically, examples of the aluminum source that can be used may include aluminum nitrate, aluminum chloride, and sodium aluminate. Among these aluminum sources, aluminum nitrate or sodium aluminate is preferable.

As for the templating agent, a conventionally known templating agent used for synthesis of the ZSM-5 zeolite may be used. Specifically, examples of the templating agent that can be used may include a tetrapropylammonium salt, a tetraethylammonium salt, propanolamine, ethanolamine, n-propylamine, morpholine, 1,5-diaminopentane, 1,6-diaminohexane, dipropylenetetramine, and triethylenetetramine. Among these templating agents, a tetrapropylammonium salt is preferable.

Examples of the alkali metal source that can be used may include a hydroxide that contains an alkali metal, a chloride that contains an alkali metal, a bromide that contains an alkali metal, and a sulfide that contains an alkali metal. Examples of the alkali metal may include sodium and potassium.

When the alkali metal is sodium, the sodium source is a compound containing sodium (Na). Specific examples of the sodium-containing compound as the sodium source may include sodium hydroxide, sodium chloride, sodium bromide, sodium sulfate, sodium silicate, sodium aluminate, as well as a compound containing sodium as a counter cation.

When the alkali metal is potassium, the potassium source is a compound containing potassium (K). Specific examples of the potassium (K)-containing compound as the potassium source may include potassium hydroxide, potassium chloride, potassium bromide, potassium sulfate, potassium silicate, potassium aluminate, as well as a compound containing potassium as a counter cation.

The ratio of the mole number of the silicon atoms to the mole number of the aluminum atoms in the mixture described above is preferably in the range of 40 to 800, and more preferably in the range of 80 to 250.

Also, as for the ratio of the mole number of the mixture described above to the mole number of the silicon atoms, preferably the followings are satisfied.

    Templating agent: 0.02 or more, and 0.4 or less,
    Alkali source: 0.02 or more, and 0.4 or less, and
    Water: 2 or more, and 100 or less

Furthermore, as for the ratio of the mole number of the mixture described above to the mole number of the silicon atoms, more preferably the followings are satisfied.

    Templating agent: 0.05 or more, and 0.3 or less,
    Alkali source: 0.04 or more, and 0.3 or less, and
    Water: 5 ore more, and 50 or less

The mixture described above is crystallized in a sealed pressure vessel by heating in the temperature range of 100°C to 200°C for 1 to 120 hours to prepare the ZSM-5 zeolite, which is a precursor to the MFI zeolite to be used in the embodiment. More specifically, after the crystallization is over, the crystals are allowed to be sufficiently cooled, followed by solid-liquid separation. Then, this solid is washed with a sufficient amount of purified water, followed by drying in the temperature range of 100°C to 150°C to obtain the ZSM-5 zeolite. Thereafter, this may be calcinated further in the temperature range of about 300°C to about 600°C.

In the ZSM-5 zeolite prepared as described above, there is a relatively large amount of sodium or potassium, which has been used as the alkali source. Therefore, usually it is necessary to remove the alkali source contained in the ZSM-5 zeolite in order to obtain the MFI zeolite to be used in the embodiment.

One method for removing the alkali source in the ZSM-5 zeolite is, for example, to contact the ZSM-5 zeolite with an aqueous solution of an ammonium salt.

Examples of the ammonium salt may include ammonium sulfate, ammonium hydrogen sulfate, ammonium carbonate, ammonium hydrogen carbonate, diammonium hydrogen phosphate, ammonium dihydrogen phosphate, ammonium phosphate, ammonium hydrogen pyrophosphate, ammonium pyrophosphate, ammonium chloride, ammonium salts of inorganic acids such as ammonium nitrate, and ammonium salts of organic acids such as ammonium acetate. Among these, ammonium sulfate, ammonium chloride, and ammonium nitrate are preferable.

The aqueous solution of the ammonium salt and the ZSM-5 zeolite are mixed and caused to contact to each other in the temperature range of 50°C to 200°C for 1 to 48 hours, so that the ZSM-5 zeolite having the content of the alkali source reduced can be obtained. More specifically, after contacting, the mixture is allowed to be sufficiently cooled, followed by solid-liquid separation. Then, this solid is washed with a sufficient amount of purified water, followed by drying at an arbitrary temperature in the range of 60°C to 150°C, so that the ZSM-5 zeolite having the content of the alkali source reduced can be obtained. Thereafter, this may be calcinated further in the temperature range of about 300°C to about 600°C.

The MFI zeolite to be used in the embodiment is preferably the ZSM-5 zeolite having the sodium atoms introduced after the alkali source is reduced.

One method for introducing the sodium atoms is, for example, causing the ZSM-5 zeolite having the alkali source reduced, which is prepared as described above, to contact with an aqueous solution of a sodium compound.

Examples of the sodium compound may include sodium hydroxide, sodium chloride, sodium bromide, sodium sulfate, sodium nitrate, and sodium phosphate.

The ZSM-5 zeolite having the alkali source reduced, which is prepared as described above, is mixed with the aqueous solution of the sodium compound, followed by causing the components in the resulting mixture to contact to each other in the temperature range of 50°C to 200°C for 1 to 48 hours, so that the MFI zeolite to be used in the embodiment can be obtained. More specifically, after contacting, the mixture is allowed to be sufficiently cooled, followed by solid-liquid separation. Then, this solid is washed with a sufficient amount of purified water, followed by drying at an arbitrary temperature in the range of 60°C to 150°C to obtain the MFI zeolite. Thereafter, this may be calcinated further in the temperature range of about 300°C to about 600°C.

The ratio of the mole number of the silicon atoms to the mole number of the aluminum atoms in the MFI zeolite to be used in the embodiment is preferably in the range of 40 to 800, and more preferably in the range of 50 to 500.

• Contact

The contact temperature at the step (2): $T^2$ is usually in the range of 400°C to 700°C, and preferably in the range of 450°C to 600°C.

It is preferable that the pyrolysis temperature $T^1$ at the step (1) and the contact temperature $T^2$ at the step (2) satisfy the following equation.

$$0°C \leq T^2 - T^1 \leq 200°C$$

The contact pressure at the step (2): $P^2$ is usually in the range of 0 MPaG to 5 MPaG, and preferably in the range of 0 MPaG to 0.5 MPaG.

In the contact at the step (2), a water vapor, or an inert gas such as nitrogen gas or $CO_2$ gas may coexist.

The contact at the step (2) is usually carried out in a reaction vessel made of a quartz glass, a carbon steel, a stainless steel, or the like.

The olefins obtained at the step (2) are usually olefins having the carbon atom number of 2 to 5, such as ethylene, propylene, butene, and the like, and preferably the olefins having the carbon atom number of 2 to 3.

[Examples]

Hereinafter, in order to explain the present invention in more detail, Examples (Examples 1 to 5 and Comparative Example 1) will be described. The present invention is not limited to the Examples described below.

The Na content (% by mass), the Si/Al ratio (dimensionless), the yield (%) of the olefins having the carbon atom number of 2 to 3, the corrected yield (%), and the ratio of the olefins having the carbon atom number of 2 to 3 to the paraffin (dimensionless), in Examples 1 to 5 and Comparative Example 1, are summarized in Table 1 below.

<Example 1>

Preparation of Catalyst

A mixture of H+-ZSM-5 (manufactured by N.E. Chemcat Corporation) (1.0 g) and an aqueous solution of 0.1 M sodium nitrate (100 mL) was charged into a 200 mL glass beaker, and then, this was allowed to be left at a temperature of 80°C for 2 hours.

This mixture was then filtrated using a Buchner funnel to collect the residue. A mixture of the collected residue with the aqueous solution of 0.1 M sodium nitrate (100 mL) was charged into the above-mentioned glass beaker, and then, this was allowed to be left at 80°C for 2 hours.

The resulting mixture was then filtrated using a Buchner funnel to collect the residue, and then this was washed with 500 mL of purified water. The washed residue was then dried at 110°C for 24 hours, followed by calcinating at 550°C for 5 hours to obtain a catalyst A (0.71 g) in the powder form.

The obtained catalyst A was analyzed by the ICP atomic emission spectrometry, and as a result, the content of the sodium atoms was 0.18% by mass, and the ratio of the mole number of the silicon atoms to the mole number of the aluminum atoms (Si/Al) was 120.

Olefin Production

An upstream reactor tube of two glass reactor tubes connected in series was filled with polyethylene (tradename: Sumikacene G201F, manufactured by Sumitomo Chemical Co., Ltd.) (0.5 g), and a downstream reactor tube was filled with the catalyst A (0.1 g).

A cooling trap was connected in further downstream of the downstream reactor tube, and a 2-L gas bag was connected in downstream of the cooling trap.

Nitrogen gas was flowed at 10 NmL/min from the upstream side of the connected glass reactor tubes, only the downstream reactor tube was heated at 550°C for 1 hour for pretreatment of the catalyst A, and then the temperature of the downstream reactor tube was lowered to 525°C.

Pyrolysis at the step (1) was carried out by heating the upstream reactor tube from the outside thereof at 455°C while flowing nitrogen gas at 10 NmL/min to obtain decomposed products. The temperature $T^1$ at this time is estimated to be 455°C.

The catalytic cracking at the step (2) was carried out by introducing the decomposed products obtained as described above into the downstream reactor tube whose temperature $T^2$ was set to 525°C so as to cause the contact with the catalyst A.

All the liquid products obtained by the catalytic cracking in 2 hours from the start of heating of the charged polyethylene were collected in the cooling trap cooled with an iced water, and all the gaseous products by the catalytic cracking were collected in the gas bag.

The mass of the liquid products by the catalytic cracking and the mass of the residue attached to the upstream reactor tube and downstream reactor tube were measured using a balance.

The collected gaseous products by the catalytic cracking were analyzed by gas chromatography, and as a result, the yield of the olefins having the carbon atom number of 2 to 3 was 22.8% in terms of the mass of the charged polyethylene.

The analysis result of the gaseous products by the catalytic cracking was corrected such that the sum of the mass of the gaseous products by the catalytic cracking, the liquid products by the catalytic cracking, and the residue thereof would equal the mass of the charged polyethylene, and as a result, the corrected yield of the olefins having the carbon atom number of 2 to 3 was 30.1% in terms of the mass of the charged polyethylene, and the olefin/paraffin ratio was 10.6.

<Comparative Example 1>

Preparation of Catalyst

Into a 400-mL PTFE vessel equipped with a stirrer, were added tetraethyl orthosilicate (32.0 g), aluminum nitrate nonahydrate (0.385 g), an aqueous solution of 40% by mass of tetrapropylammonium hydroxide (19.5 g), sodium hydroxide (0.62 g), and ion-exchanged water (11.2 g), and then the mixture was stirred at room temperature for 24 hours. Then, after ion-exchanged water (18 g) was added, the whole of the resulting mixture was transferred to a SUS-made autoclave equipped with a 200-mL PTFE inner cylinder vessel.

In this mixture, the ratio of the mole number of the silicon atoms to the mole number of the aluminum atoms was 150.1; and to the mole number of the silicon atoms, the ratio of the mole number of the tetrapropylammonium hydroxide was 0.25, the ratio of the mole number of the sodium atoms was 0.10, and the ratio of the mole number of water was 14.8.

The mixture was heated in the autoclave at 170°C for 24 hours followed by cooling with an iced water. After cooling, the suspension solution in the cylindrical vessel was centrifuged, and the solid was collected by removing the supernatant liquid. Water was added to the thus collected solid to make a suspension solution again, and then the washed solid was obtained by removing the supernatant liquid with centrifugation. The endpoint of washing was determined when the pH of the supernatant liquid reached 8 or lower.

The resulting solid was then dried at 120°C or 8 hours. The obtained solid was crushed using a mortar, and then further calcinated using a muffle furnace at 550°C for 7 hours to obtain a catalyst B (4.6 g) in the powder form.

The obtained catalyst B was analyzed by the ICP atomic emission spectrometry, and as a result, the content of the sodium atoms was 0.56% by mass, and the ratio of the mole number of the silicon atoms to the mole number of the aluminum atoms (Si/Al) was 226.

Olefin Production

Olefin production was carried out by the method as described in Example 1, except that the catalyst B was used instead of the catalyst A in Example 1.

The collected gaseous products by the catalytic cracking were analyzed by gas chromatography, and as a result, the yield of the olefins having the carbon atom number of 2 to 3 was 4.3% in terms of the mass of the charged polyethylene.

The analysis result of the gaseous products by the catalytic cracking was corrected such that the sum of the mass of the gaseous products by the catalytic cracking, the liquid products by the catalytic cracking, and the residue thereof would equal the mass of the charged polyethylene, and as a result, the corrected yield of the olefins having the carbon atom number of 2 to 3 was 7.3% in terms of the mass of the charged polyethylene, and the olefin/paraffin ratio was 2.0.

<Example 2>

Preparation of Catalyst

The catalyst B (3 g) and an aqueous solution of 0.5 M ammonium nitrate (150 mL) were charged into a flask, and then, this was allowed to be left at a temperature of 60°C for 24 hours.

The resulting mixture was then suction filtrated using a Buchner funnel to collect the residue. To the flask were added the collected residue and the aqueous solution of 0.5 M ammonium nitrate (250 mL), and then, this was allowed to be left at a temperature of 60°C for 20 hours.

The resulting mixture was then suction filtrated using a Buchner funnel to collect the residue. To the flask were added the collected residue and the aqueous solution of 0.5 M ammonium nitrate (150 mL), and then, this was allowed to be left at a temperature of 60°C for 6 hours.

The resulting mixture was then suction filtrated using a Buchner funnel to collect the residue. The collected residue was washed with water (500 mL), and the resulting solid was dried at 90°C for 12 hours. The obtained solid was calcinated using a muffle furnace at a temperature of 550°C for 5 hours to obtain a solid 1 (2.1 g).

Into a two-necked 200-mL glass round-bottom flask equipped with a cooling tube, were added the solid 1 (1 g) and an aqueous solution of 0.1 M sodium nitrate (100 mL), and then, this was allowed to be left at a temperature of 100°C for 2 hours.

The resulting mixture was then suction filtrated using a Buchner funnel to collect the residue. To the above round-bottom flask, were added the collected residue and the aqueous solution of 0.1 M sodium nitrate (100 mL), and then, this was allowed to be left at a temperature of 100°C for 2 hours.

The resulting mixture was then suction filtrated using a Buchner funnel to collect the residue. The collected residue was dried at a temperature of 110°C for 12 hours to obtain a catalyst C (0.9 g) in the powder form.

The obtained catalyst C was analyzed by the ICP atomic emission spectrometry, and as a result, the content of the sodium atoms was 0.23% by mass, and the ratio of the mole number of the silicon atoms to the mole number of the aluminum atoms (Si/Al) was 226.

Olefin Production

Olefin production was carried out by the method as described in Example 1, except that the catalyst C was used instead of the catalyst A in Example 1.

The collected gaseous products by the catalytic cracking were analyzed by gas chromatography, and as a result, the yield of the olefins having the carbon atom number of 2 to 3 was 16.8% in terms of the mass of the charged polyethylene.

The analysis result of the gaseous products by the catalytic cracking was corrected such that the sum of the mass of the gaseous products by the catalytic cracking, the liquid products by the catalytic cracking, and the residue thereof would equal the mass of the charged polyethylene, and as a result, the corrected yield of the olefins having the carbon atom number of 2 to 3 was 23.6% in terms of the mass of the charged polyethylene, and the olefin/paraffin ratio was 10.1.

<Example 3>

Preparation of Catalyst

Into a 400-mL PTFE vessel equipped with a stirrer, were added tetraethyl orthosilicate (32.0 g), aluminum nitrate nonahydrate (0.576 g), an aqueous solution of 20.3% by mass of tetrapropylammonium hydroxide (38.4 g), and sodium hydroxide (0.384 g), and then, the mixture was stirred at room temperature for 24 hours.

Then, after ion-exchanged water (28 g) was added, the whole of the resulting mixture was transferred to a SUS-made autoclave equipped with a 200-mL PTFE inner cylinder vessel.

In this mixture, the ratio of the mole number of the silicon atoms to the mole number of the aluminum atoms was 100.1; and the ratios of the mole numbers of the tetrapropylammonium hydroxide, of the sodium atoms, and of the water, to the mole number of the silicon atoms, were 0.25, 0.06, and 21.3, respectively.

The mixture was heated in the autoclave at 170°C for 24 hours followed by cooling with an iced water. After cooling, the suspension solution in the cylinder vessel was centrifuged, and the solid was collected by removing the supernatant liquid.

Water was added to the thus collected solid to make a suspension solution again, and then the washed solid was obtained by removing the supernatant liquid with centrifugation. The endpoint of washing was determined when the pH of the supernatant liquid reached 8 or lower.

The collected solid was then dried at a temperature of 120°C for 8 hours. The obtained solid was crushed using a mortar, and then further calcinated using a muffle furnace at a temperature of 550°C for 7 hours to obtain a solid 2 (12.7 g).

Into a flask, were added the solid 2 (5.0 g) and an aqueous solution of 0.5 M ammonium nitrate (250 mL), and then, this was allowed to be left at a temperature of 60°C for 6 hours. The resulting mixture was then suction filtrated using a Buchner funnel to collect the residue.

To the flask were added the collected residue and the aqueous solution of 0.5 M ammonium nitrate (250 mL), and then, this was allowed to be left at a temperature of 60°C for 12 hours. The resulting mixture was then suction filtrated using a Buchner funnel to collect the residue.

To the flask were added the collected residue and the aqueous solution of 0.5 M ammonium nitrate (250 mL), and then, this was allowed to be left at a temperature of 60°C for 6 hours. The resulting mixture was then suction filtrated using a Buchner funnel to collect the residue.

The collected residue was washed with water (500 mL), and the resulting solid was dried at a temperature of 90°C for 6 hours. The obtained solid was calcinated using a muffle furnace at a temperature of 550°C for 5 hours to obtain a solid 3 (3.7 g).

Into a two-necked 500-mL glass round-bottom flask equipped with a cooling tube, were added the solid 3 (3 g) and an aqueous solution of 0.1 M sodium nitrate (300 mL), and then, this was allowed to be left at a temperature of 100°C for 2 hours.

The resulting mixture was then suction filtrated using a Buchner funnel to collect the residue. To the above round-bottom flask, were added the collected residue and the aqueous solution of 0.1 M sodium nitrate (300 mL), and then, this was allowed to be left at a temperature of 100°C for 2 hours.

The resulting mixture was then suction filtrated using a Buchner funnel to collect the residue. The collected residue was dried at a temperature of 100°C for 24, followed by baking at a temperature of 550°C for 5 hours to obtain a catalyst D (2.1 g) in the powder form.

The obtained catalyst D was analyzed by the ICP atomic emission spectrometry, and as a result, the content of the sodium atoms was 0.21% by mass, and the ratio of the mole number of the silicon atoms to the mole number of the aluminum atoms (Si/Al) was 94.

Olefin Production

Olefin production was carried out by the method as described in Example 1, except that the catalyst D was used instead of the catalyst A in Example 1. The collected liquid and gaseous products by the catalytic cracking were respectively analyzed by gas chromatography, and as a result, the yield of the olefins having the carbon atom number of 2 to 3 was 20.7% in terms of the mass of the charged polyethylene.

Moreover, the analysis result of the gaseous products by the catalytic cracking was corrected such that the sum of the mass of the gaseous products by the catalytic cracking, the liquid products by the catalytic cracking, and the residue thereof would equal the mass of the charged polyethylene, and as a result, the corrected yield of the olefins having the carbon atom number of 2 to 3 was 23.9% in terms of the mass of the charged polyethylene, and the olefin/paraffin ratio was 12.1.

<Example 4>

Preparation of Catalyst

Into a 400-mL PTFE vessel equipped with a stirrer, were added tetraethyl orthosilicate (64.0 g), aluminum nitrate nonahydrate (1.152 g), an aqueous solution of 20.3% by mass of tetrapropylammonium hydroxide (76.8 g), and sodium hydroxide (0.768 g), and then, the mixture was stirred at room temperature for 24 hours.

Then, after ion-exchanged water (57 g) was added, the whole of the resulting mixture was transferred to a SUS-made autoclave equipped with a 200-mL PTFE inner cylinder vessel.

In this mixture, the ratio of the mole number of the silicon atoms to the mole number of the aluminum atoms was 100.0; and the ratios of the mole numbers of the tetrapropylammonium hydroxide, of the sodium atoms, and of the water, to the mole number of the silicon atoms, were 0.25, 0.06, and 21.3, respectively.

The mixture was heated in the autoclave at a temperature of 170°C for 24 hours followed by cooling with an iced water. After cooling, the suspension solution in the cylinder vessel was centrifuged, and the solid was collected by removing the supernatant liquid.

Water was added to the thus collected solid to make a suspension solution again, and then the washed solid was obtained by removing the supernatant liquid with centrifugation. The endpoint of washing was determined when the pH of the supernatant liquid reached 8 or lower.

The collected solid was then dried at a temperature of 120°C for 8 hours. The obtained solid was crushed using a mortar, and then further calcinated using a muffle furnace at a temperature of 550°C for 7 hours to obtain a solid 4 (13.6 g).

Into a flask, were added the solid 4 (6.0 g) and an aqueous solution of 0.5 M ammonium nitrate (300 mL), and then, this was allowed to be left at a temperature of 60°C for 6 hours. The resulting mixture was then suction filtrated using a Buchner funnel to collect the residue.

To the flask, were added the collected residue and the aqueous solution of 0.5 M ammonium nitrate (300 mL), and then, this was allowed to be left at a temperature of 60°C for 12 hours.

The resulting mixture was then suction filtrated using a Buchner funnel to collect the residue. To the flask were added the collected residue and the aqueous solution of 0.5 M ammonium nitrate (250 mL), and then, this was allowed to be left at a temperature of 60°C for 6 hours.

The resulting mixture was then suction filtrated using a Buchner funnel to collect the residue. The collected residue was washed with water (500 mL), and the resulting solid was dried at a temperature of 90°C for 6 hours. The obtained solid was calcinated using a muffle furnace at a temperature of 550°C for 5 hours to obtain a solid 5 (4.6 g).

Into a two-necked 500 mL glass-made round-bottle flask equipped with a cooling tube, were added the solid 5 (4.5 g) and an aqueous solution of 0.1 M sodium nitrate (450 mL), and then, this was allowed to be left at a temperature of 100°C for 2 hours.

The resulting mixture was then filtrated using a Buchner funnel to collect the residue. To the above round-bottom flask, were added the collected residue and the aqueous solution of 0.1 M sodium nitrate (450 mL), and then, this was allowed to be left at a temperature of 100°C for 2 hours.

The resulting mixture was then suction filtrated using a Buchner funnel to collect the residue. The collected residue was then dried at a temperature of 110°C for 12 hours, followed by baking at a temperature of 550°C for 5 hours to obtain a catalyst E (4.1 g) in the powder form.

The obtained catalyst E was analyzed by the ICP atomic emission spectrometry, and as a result, the content of the sodium atoms was 0.24% by mass, and the ratio of the mole number of the silicon atoms to the mole number of the aluminum atoms (Si/Al) was 85.

Olefin Production

The upstream reactor tube of two glass reactor tubes connected in series was filled with polypropylene (tradename: Noblen FS2011DG3 manufactured by Sumitomo Chemical Co., Ltd.) (1.0 g), and the downstream reactor tube was filled with the catalyst E (0.2 g). A cooling trap was connected in further downstream of the downstream reactor tube, and a 2-L gas bag was connected in downstream of the cooling trap.

Nitrogen gas was flowed at 10 NmL/min from the upstream side of the connected glass reactor tubes, only the downstream reactor tube was heated at 550°C for 1 hour for pretreatment of the catalyst E, and then the temperature of the downstream reactor tube was lowered to 525°C. Pyrolysis at the step (1) was carried out by heating the upstream reactor tube from the outside thereof at 415°C while flowing nitrogen gas at 10 NmL/min to obtain decomposed products. The temperature $T^1$ at this time is estimated to be 445°C.

The catalytic cracking at the step (2) was carried out by introducing the decomposed products obtained as described above into the downstream reactor tube whose temperature $T^2$ was set to 525°C so as to cause the contact with the catalyst E.

All the liquid products obtained by the catalytic cracking in 2 hours from the start of heating of the charged polypropylene were collected in the cooling trap cooled with an iced water, and all the gaseous products obtained by the catalytic cracking were collected in the gas bag.

The mass of the liquid products by the catalytic cracking and the mass of the residue attached to the upstream reactor tube and downstream reactor tube were measured using a balance. The collected gaseous products by the catalytic cracking were analyzed by gas chromatography, and as a result, the yield of the olefins having the carbon atom number of 2 to 3 was 16.7% in terms of the mass of the charged polypropylene.

The analysis result of the gaseous products by the catalytic cracking was corrected such that the sum of the mass of the gaseous products by the catalytic cracking, the liquid products by the catalytic cracking, and the residue thereof would equal the mass of the charged polypropylene, and as a result, the corrected yield of the olefins having the carbon atom number of 2 to 3 was 19.8% in terms of the mass of the charged polypropylene, and the olefin/paraffin ratio was 12.5.

<Example 5>

Olefin Production

The upstream reactor tube of two glass reactor tubes connected in series was filled with a mixture of polyethylene (tradename: Sumikacene G201F, manufactured by Sumitomo Chemical Co., Ltd.) (0.5 g) and polypropylene (tradename: Sumikacene G201F, manufactured by Sumitomo Chemical Co., Ltd.) (0.5 g), and the downstream reactor tube was filled with the catalyst D (0.2 g).

A cooling trap was connected in further downstream of the downstream reactor tube, and a 2-L gas bag was connected in downstream of the cooling trap. Nitrogen gas was flowed at 10 NmL/min from the upstream of the reactor tubes, only the downstream reactor tube was heated at 550°C for 1 hour for pretreatment of the catalyst D, and then the temperature of the downstream reactor tube was lowered to 525°C.

Pyrolysis at the step (1) was carried out by heating the upstream reactor tube from the outside thereof at 455°C while flowing nitrogen gas at 10 NmL/min to obtain decomposed products. The temperature: $T^1$ at this time is estimated to be 445°C.

The catalytic cracking at the step (2) was carried out by introducing the decomposed products obtained as described above into the downstream reactor tube whose temperature $T^2$ was set to 525°C so as to cause the contact with the catalyst E.

All the liquid products obtained by the catalytic cracking in 2 hours from the start of heating of the charged mixture of polyethylene and polypropylene were collected in the cooling trap cooled with an iced water, and all the gaseous products by the catalytic cracking were collected in the gas bag.

The mass of the liquid products by the catalytic cracking and of the residue attached to the upstream reactor tube and downstream reactor tube were measured using a balance. The collected liquid and gaseous products by the catalytic cracking were respectively analyzed by gas chromatography, and as a result, the yield of the olefins having the carbon atom number of 2 to 3 was 23.2% in terms of the mass of the charged mixture of polyethylene and polypropylene.

The analysis result of the gaseous products by the catalytic cracking was corrected such that the sum of the mass of the gaseous products by the catalytic cracking, the liquid products by the catalytic cracking, and the residue thereof would equal the total mass of the charged mixture of polyethylene and polypropylene, and as a result, the corrected yield of the olefins having the carbon atom number of 2 to 3 was 32.9% in terms of the mass of the charged mixture of polyethylene and polypropylene, and the olefin/paraffin ratio was 6.2.

[Table 1]

|  | Example 1 | Example 2 | Example 3 | Example 4 | Example 5 | Comparative Example 1 |
|---|---|---|---|---|---|---|
| Na content (% by mass) | 0.18 | 0.23 | 0.21 | 0.24 | 0.21 | 0.56 |
| Si/Al ratio (-) | 120 | 226 | 94 | 85 | 94 | 226 |
| Polyolefin | Polyethylene | Polyethylene | Polyethylene | Polypropylene | Polyethylene/ Polypropylene =1/1 (ratio by mass) | Polyethylen e |

(continued)

|  | Example 1 | Example 2 | Example 3 | Example 4 | Example 5 | Comparative Example 1 |
|---|---|---|---|---|---|---|
| Yield of the olefins having the carbon atom number of 2 to 3(%) | 22.8 | 16.8 | 20.7 | 16.7 | 23.2 | 4.3 |
| Corrected yield(%) | 30.1 | 23.6 | 23.9 | 19.8 | 32.9 | 7.3 |
| Olefin having the carbon atom number of 2 to 3/paraffin ratio (-) | 10.6 | 10.1 | 12.1 | 12.5 | 6.2 | 2.0 |

**Claims**

1. A method for producing olefins comprising following steps (1) and (2):

   step (1): heating a polyolefin plastic to obtain a decomposed product (pyrolysis step); and
   step (2): causing the decomposed product obtained at the step (1) to contact with an MFI zeolite containing sodium atoms in the range of 0.10% by mass to 0.30% by mass to obtain catalytically cracked products containing olefins (catalytic cracking step).

2. The method for producing olefins according to claim 1, wherein the olefins are olefins having the carbon atom number of 2 to 3.

3. The method for producing olefins according to claim 1 or 2, wherein a ratio of a mole number of silicon atoms to a mole number of aluminum atoms in the MFI zeolite is 80 to 250.

4. The method for producing olefins according to any one of claims 1 to 3, wherein a pyrolysis temperature $T^1$ at the step (1) is 400°C to 500°C.

5. The method for producing olefins according to any one of claims 1 to 4, wherein a contact temperature $T^2$ at the step (2) is 450°C to 600°C.

6. The method for producing olefins according to any one of claims 1 to 5, wherein a pyrolysis temperature $T^1$ at the step (1) and a contact temperature $T^2$ at the step (2) satisfy a following equation:

$$0°C \leq T^2 - T^1 \leq 200°C.$$

7. The method for producing olefins according to any one of claims 1 to 6, wherein the polyolefin plastic is at least one plastic selected from the group consisting of polyethylene and polypropylene.

8. The method for producing olefins according to claim 7, wherein the polyolefin plastic is a mixture of polyethylene and polypropylene.

## INTERNATIONAL SEARCH REPORT

| | International application No. |
|---|---|
| | PCT/JP2021/005557 |

### A. CLASSIFICATION OF SUBJECT MATTER

Int.Cl. C07C4/00(2006.01)i, B01J29/40(2006.01)i, C01B39/38(2006.01)i,
C07B61/00(2006.01)i, C07C11/04(2006.01)i, C07C11/06(2006.01)i
FI: C07C4/00, C07C11/04, C07C11/06, B01J29/40Z, C01B39/38, C07B61/00300

According to International Patent Classification (IPC) or to both national classification and IPC

### B. FIELDS SEARCHED

Minimum documentation searched (classification system followed by classification symbols)
Int.Cl. C07C4/00, B01J29/40, C01B39/38, C07B61/00, C07C11/04, C07C11/06

Documentation searched other than minimum documentation to the extent that such documents are included in the fields searched
Published examined utility model applications of Japan    1922-1996
Published unexamined utility model applications of Japan   1971-2021
Registered utility model specifications of Japan           1996-2021
Published registered utility model applications of Japan   1994-2021

Electronic data base consulted during the international search (name of data base and, where practicable, search terms used)
JSTPlus(JDreamIII), JST7580(JDreamIII), JSTChina(JDreamIII)

### C. DOCUMENTS CONSIDERED TO BE RELEVANT

| Category* | Citation of document, with indication, where appropriate, of the relevant passages | Relevant to claim No. |
|---|---|---|
| X | 加賀慎之介 他, ポリエチレンの接触分解による低級オレフィン化, 石油学会年会・秋季大会講演要旨集, 2017, 第47回石油・石油化学討論会 (鳥取), 190, https://doi.org/10.11523/sekiyu.2017f.0_163, session ID 2D23, p. 190, 2. experiments, 3. results and considerations, table 1, (KAGA, Shinnosuke et al., Catalytic degradation of polyethylene into lower olefins, Proceeding of Annual/Fall Meetings of the Japan Petroleum Institute 2017), non-official translation (47th Petroleum and Petrochemical Conference (Tottori)) | 1-8 |

☒ Further documents are listed in the continuation of Box C.   ☒ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 10 March 2021 | 30 March 2021 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.

PCT/JP2021/005557

C (Continuation).   DOCUMENTS CONSIDERED TO BE RELEVANT

| Category* | Citation of document, with indication, where appropriate, of the relevant passages | Relevant to claim No. |
|---|---|---|
| X | 上道芳夫 他，ポリオレフィンの接触分解による低級オレフィン化，ファインケミカル, 2017, 46(12), 44-51, pp. 46-49, table 1, fig. 5, (KAMIMICHI, Yoshio et al., Catalytic Degradation of Polyolefins into Lower Olefins, Fine Chemical) | 1-8 |
| A | WO 2007/080957 A1 (ASAHI KASEI CHEMICALS CORPORATION) 19 July 2007 (2007-07-19), paragraph [0045] | 1-8 |

Form PCT/ISA/210 (second sheet) (January 2015)

| INTERNATIONAL SEARCH REPORT | International application No. |
|---|---|
| Information on patent family members | PCT/JP2021/005557 |

```
WO 2007/080957 A1  19 July 2007  US 2010/0022810 A1
                                  paragraph [0091]
                                  EP 1975143 A1
                                  CN 101370755 A
                                  KR 10-2008-0081288 A
```

Form PCT/ISA/210 (patent family annex) (January 2015)